(19) Europäisches Patentamt
European Patent Office
Office européen des brevets

(11) Veröffentlichungsnummer: **0 057 424**
**A1**

# (12) EUROPÄISCHE PATENTANMELDUNG

(21) Anmeldenummer: **82100566.7**

(22) Anmeldetag: **28.01.82**

(51) Int. Cl.³: **C 07 C 103/28**, C 07 C 103/76, C 07 C 102/00

(30) Priorität: **03.02.81 DE 3103563**

(43) Veröffentlichungstag der Anmeldung: **11.08.82**
**Patentblatt 82/32**

(84) Benannte Vertragsstaaten: **BE CH DE FR GB LI**

(71) Anmelder: **BASF Aktiengesellschaft,**
**Carl-Bosch-Strasse 38, D-6700 Ludwigshafen (DE)**

(72) Erfinder: **Tonne, Peter, Dr., Triftbrunnenweg 63,**
**D-6730 Neustadt (DE)**
Erfinder: **Ludwig, Winfried, Dr., Oberer Bergelweg 3,**
**D-6718 Gruenstadt (DE)**
Erfinder: **Kilpper, Gerhard, Dr., Am Tannenhang 1,**
**D-6719 Carlsberg (DE)**
Erfinder: **Grimmer, Johannes, Duererstrasse 12,**
**D-6700 Ludwigshafen (DE)**

(54) **Verfahren zur Herstellung von Anthranilsäureamiden.**

(57) Herstellung von Anthranilsäureamiden durch Vermischen von Phthalamidsäure mit Lauge, Katalysator und Hypohalogenit, wobei man anschliessend das Gemisch während 1 bis 1 000 Sekunden stehen lässt, nun das Amin und darauf Säure bis zu einem pH-Wert von 6 bis 8 in das Gemisch eingibt.

Die nach dem Verfahren der Erfindung herstellbaren Anthranilsäureamide sind wertvolle Ausgangsstoffe für die Herstellung von Farbstoffen, Riechstoffen und Schädlingsbekämpfungsmitteln.

EP 0 057 424 A1

Verfahren zur Herstellung von Anthranilsäureamiden

Die Erfindung betrifft ein neues Verfahren zur Herstellung von Anthranilsäureamiden durch Vermischen von Phthalamidsäure mit Lauge, Katalysator und Hypohalogenit, wobei man anschließend das Gemisch während 1 bis 1 000 Sekunden stehen läßt, nun das Amin und darauf Säure bis zu einem pH-Wert von 6 bis 8 in das Gemisch eingibt.

Herstellverfahren, die auf der Umsetzung von Isatosäureanhydrid mit Aminen beruhen, sind bekannt.

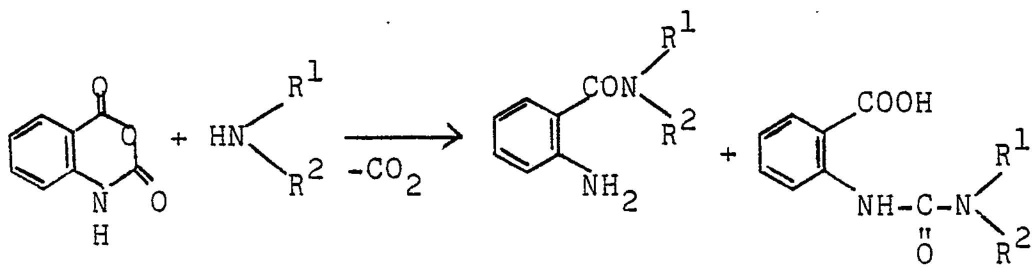


IV

Dabei entstehen jedoch nicht nur die gewünschten Anthranilamide, sondern in beträchtlichem Umfang als Nebenprodukte Harnstoffderivate IV, die die Ausbeute vermindern und nachträglich Reinigungsoperationen erforderlich machen (J. Am. Chem. Soc. 88 (1966), 4001 - 4008).

Es ist aus J. prakt. Chem., [2] 30, 467 ff (1884); J. org.Chem. 13, 347 ff (1948), 24, 1214 ff (1959) bekannt, daß sich Isatosäureanhydrid mit wäßrigem Ammoniak zu Anthranilsäureamid und o-Ureidobenzoesäure umsetzt. Die Umsetzung von 5,7 Dichlor-isatosäureanhydrid mit Ammoniak führt zu 6,8-Dichlorbenzoylharnstoff (J.org.Chem. 3, 414 ff (1938) Band 12, 743 (1947)), andere durch Chloratome substituierte Isatosäureanhydride ergeben nur mäßige Ausbeuten an chlorierten Anthranilsäureamiden (J.org.Chem. 26, 613 ff (1961). Eine entsprechende Reaktion des 6-Nitroisatosäure-

WB/P

anhydrids, wie sie eine Arbeit in J.prakt.Chem., [2] 30, 467 ff (1884) beschreibt, liefert 5-Nitroanthranilsäureamid in schlechter Ausbeute und Reinheit. Von dem so erhaltenen Endstoff konnte nur ein Zersetzungspunkt erhalten werden, der ca 30°C unterhalb des Schmelzpunktes des reinen Endstoffs liegt. Das offenbar durch Nitroureidobenzoesäure verunreinigte Produkt läßt sich nur aufwendig, z.B. durch Salzbildung mit Salz- oder Schwefelsäure und nachfolgende Ausfällung mit Alkalilauge, abtrennen und reinigen; daher kommt dieses Verfahren insbesondere für eine großtechnische Herstellung nicht in Frage.

Es sind deshalb zahlreiche Versuche unternommen worden, um die Ausbeute an den Amiden zu verbessern. Nach dem Verfahren in DE-OS 15 43 332 wird die Umsetzung bevorzugt in einem mit Wasser mischbaren organischen Lösungsmittel ausgeführt. Dieses Verfahren weist den Nachteil auf, daß zur Isolierung der Anthranilamide in einem weiteren Verfahrensschritt entweder das Lösungsmittel abdestilliert oder aber zusätzlich mehr Wasser zugesetzt werden muß, was zu einem Verlust an eingesetztem Lösungsmittel führt. Isatosäureanhydrid und Amin werden gleichzeitig in stöchiometrischen Mengen in das Lösungsmittel gegeben.

In J.Am.Chem.Soc. 88 (1966), 4001 wird die Kinetik der Umsetzung von Isatosäureanhydrid mit Aminen an verschiedenen Beispielen untersucht. Danach ist es zweckmäßig, zur Erzielung guter Ausbeuten an Amiden in starker Verdünnung zu arbeiten (bis $10^{-3}$ M); weiterhin begünstigen höhere Aminkonzentrationen die Bildung der Harnstoffsäuren IV. Es werden deshalb Zusätze von Aminhydrochloriden und bestimmten Mengen Natriumchlorid vorgenommen. Weiterhin werden zum Teil sehr große Überschüsse an Amin/Aminhydrochlorid (z.T. mehr als 1 zu 10) verwendet. Aus diesen Gründen ist das Verfahren zur Durchführung im technischen Maßstab nicht geeignet.

In DE-OS 27 19 020 wird die Umsetzung von Isatosäureanhydrid mit Aminen vorteilhaft in einem pH-Bereich von 7 bis 10,5 vorgenommen, wobei zweckmäßig Puffersysteme aus Aminhydrochloriden und -carbonaten zur Einstellung des pH-Wertes verwendet werden. Dieses Verfahren hat den Nachteil, daß Gemische aus Amin, Aminhydrochlorid und -carbonaten verwendet werden, die in einer vorgeschalteten Stufe hergestellt werden müssen.

Es wurde nun gefunden, daß man Anthranilsäureamide der Formel

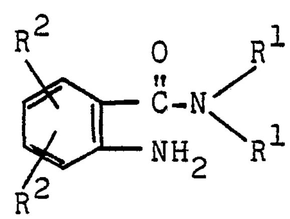

I,

worin die einzelnen Reste $R^1$ und $R^2$ gleich oder verschieden sein können und jeweils Wasserstoff oder einen aliphatischen Rest bedeuten, $R^1$ auch einen cycloaliphatischen, araliphatiscnen, aromatiscnen Rest bezeichnet, $R^2$ auch für ein Halogenatom oder eine Alkoxygruppe stehen kann, vorteilhaft erhält, wenn man Phthalamidsäuren der Formel

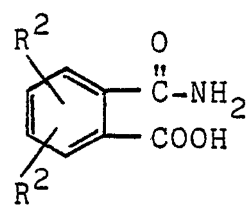

II,

worin $R^2$ die vorgenannte Bedeutung besitzt, mit Hypohalogenit und Ammoniak oder Aminen der Formel

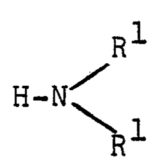

III,

worin $R^1$ die vorgenannte Bedeutung besitzt, umsetzt, wobei man den Ausgangsstoff II in wäßriger Lauge löst, mit Amidosulfonsäure, Sulfamid und/oder Kaliumiodid als Katalysator und Hypohalogenit mischt, die Mischung 1 bis 1 000 Sekunden stehen läßt und nun das Amin und darauf Säure bis zu einem pH-Wert von 6 bis 8 in das Gemisch eingibt.

Die Umsetzung läßt sich für den Fall der Verwendung von Phthalamidsäure, Ammoniak, Natronlauge und von Natriumhypochlorit durch die folgenden Formeln wiedergeben:

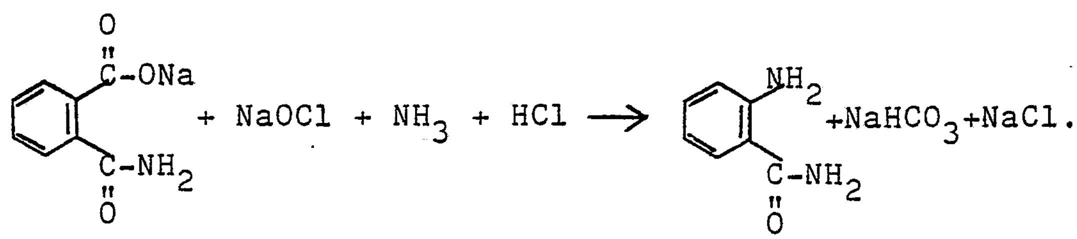

Im Vergleich zu den bekannten Verfahren liefert das Verfahren nach der Erfindung Anthranilsäureamide auf einfacherem und wirtschaftlicherem Wege in besserer Ausbeute und Reinheit und ermöglicht die Herstellung einer großen Zahl von Aminen mit Alkalihypohalogenitlösungen auch im großtechnischen Maßstab. Da die alkalische Hypochloritlösung beständiger als die Hypobromitlösung ist und ihr Gehalt auch nach Tagen kaum abnimmt, ist das erfindungsgemäße Verfahren betriebssicherer, störungsfreier und gerade auch für den industriellen Betrieb geeignet. Im Vergleich zu den bekannten Verfahren, die Hypochloridlösungen verwenden, z.B. für die Herstellung aromatischer Amine, liefert es bessere Gesamtergebnisse mit Bezug auf Ausbeute oder Reinheit des Endstoffs. Im Hinblick auf den Stand der Technik sind die vorteilhaften Ergebnisse des erfindungsgemäßen Verfahrens überraschend: Denn es ist bekannt, daß bei der Umsetzung von Carbonamiden mit Hypohalogeniten unter Zugabe von Aminen in hoher Ausbeute die entsprechenden Harnstoffe gebildet werden, z.B. siehe DE-OS 24 00 111

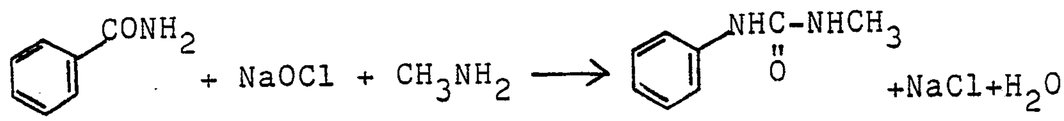

oder DE-OS 21 29 200

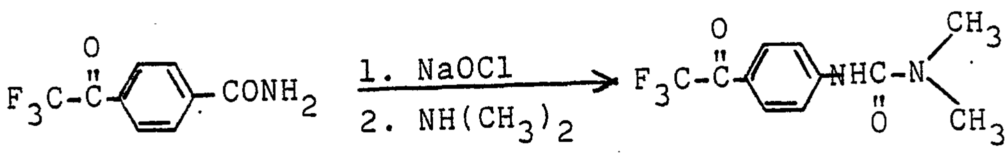


Ammoniak wird im allgemeinen in Gestalt einer wäßrigen, vorzugsweise 1- bis 25-gewichtsprozentigen Lösung, die Ausgangsstoffe II und III in flüssiger Form oder als wäßrige Suspension zugeführt.

Bevorzugte Phthalamidsäuren II und Amine III und dementsprechend bevorzugte Anthranilsäureamide I sind solche, in deren Formeln die einzelnen Reste $R^1$ und $R^2$ gleich oder verschieden sein können und jeweils Wasserstoff oder einen Alkylrest mit 1 bis 10, insbesondere 1 bis 4 Kohlenstoffatomen bedeuten, $R^1$ auch einen Cyclohexylrest, einen Aralkylrest oder Alkylarylrest mit 7 bis 12 Kohlenstoffatomen, einen Phenylrest, einen Benzoylphenylrest oder einen Naphthylrest bezeichnet, $R^2$ auch für ein Brom- oder Chloratom oder eine Alkoxygruppe mit 1 bis 4 Kohlenstoffatomen stehen kann. Die vorgenannten Reste können noch durch unter den Reaktionsbedingungen inerte Gruppen und/oder Atome, z.B. einen Benzolkern substituierende Chloratome, Bromatome oder Alkoxygruppen mit jeweils 1 bis 4 Kohlenstoffatomen; Alkylgruppen mit 1 bis 4 Kohlenstoffatomen, Nitrogruppen, substituiert sein.

Beispielsweise sind folgende Phthalamidsäuren als Ausgangsstoffe II geeignet: Phthalamidsäure; in 4-, 5-, 6-, 7-Stellung einfach durch Chlor-, Brom-atome, Methoxy-, Ethoxy-, Propoxy-, Isopropoxy-, Butoxy-, Isobutoxy-, sek.-Butoxy-, tert.-Butoxy-, Pentoxy-, Methyl-, Ethyl-, Propyl-, Isopropyl-, Butyl-, Isobutyl-, sek.-Butyl-, tert.-Butyl-, Pentyl-, Hexyl-, Hexoxy-gruppen substituierte Phthalamidsäuren.

Als Ausgangsstoffe III kommen z.B. in Betracht: Dimethylamin, Diethylamin, Dipropylamin, Diisopropylamin, Dibutylamin, Diisobutylamin, Ammoniak, Di-sek.-butylamin, Di-tert.-butylamin, Dibenzylamin, Dicyclohexylamin, Diamylamin, Dihexylamin, N-Methylanilin, N-Ethylanilin, N-Propylanilin, N-Methyltoluidin, N-Ethyltoluidin, N-Propyltoluidin, N-Methylaminoethanol, N-Ethylaminoethanol, N-Propylaminoethanol, N,N'-Dimethylethylendiamin, N,N'-Diethylethylendiamin, N-Methylcyclohexylamin; Methylamin, Ethylamin, Propylamin, Isopropylamin, Butylamin, sek.-Butylamin, tert.-Butylamin, Benzylamin, Hexylamin, Cyclohexylamin, Amylamin, Anilin, Toluidin, Aminoäthanol.

Als weitere Ausgangsstoffe verwendet man Hypohalogenite, zweckmäßig Hypochlorite in wäßrigem Medium, in der Regel in Gestalt von entsprechenden wäßrigen, alkalischen Lösungen. Vorteilhaft gelangen wäßrige Suspensionen von 1 bis 50 Gew.% Ausgangsstoff II zur Anwendung. Die wäßrigen Hypochloritlösungen enthalten im allgemeinen von 5 bis 15, vorzugsweise von 12 bis 14 Gew.% Hypochlorit und können zusätzlich bis 0,2 Mol Alkalihydroxid je Mol Hypochlorit enthalten. Im Ausgangsgemisch beider Ausgangsstoffe kommen im allgemeinen Mengen von insgesamt 0,9 bis 1,5, vorzugsweise 0,95 bis 1,1 Mol Hypochlorit und zweckmäßig von insgesamt 1,0 bis 1,5 Mol, vorzugsweise 1,0 bis 1,2 Mol Alkalihydroxid (nicht eingerechnet das im Hypochlorit enthal-

tene Alkali), bezogen auf 1 Mol Ausgangsstoff II, in Frage. Bevorzugte Alkalihypochlorite sind das Natrium- oder das Kaliumsalz.

Man setzt in der Regel bei einer Temperatur zwischen -10°C und +100°C, vorzugsweise zwischen 10 und 85°C, drucklos oder unter Druck, kontinuierlich oder diskontinuierlich um. Die Reaktion kann wie folgt durchgeführt werden: Ausgangsstoff II wird in wäßriger Lauge, vorzugsweise Natronlauge oder Kalilauge, zweckmäßig in 1- bis 50-gewichtsprozentiger wäßriger Laugelösung, gelöst. Vorteilhaft setzt man den Katalysator schon bei dieser Lösung dazu. Zweckmäßig ist ein Molverhältnis von 0,001 bis 0,2, insbesondere von 0,01 bis 0,1 Mol Katalysator pro Mol Alkalihydroxid. Nun wird wäßriges Natriumhypohalogenit zugegeben. Die Verweilzeit vom Ende der Herstellung dieses Ausgangsgemisches, wobei alle Komponenten völlig zugesetzt sind, bis zu Beginn der Zugabe des Amins III beträgt 1 bis 1 000, bevorzugt 1 bis 100 Sekunden. Die Verweilzeit zwischen Ende der Herstellung des Ausgangsgemisches und dem Zugabebeginn der Säure 1 bis 1 000, bevorzugt 1 bis 100 Sekunden. Vorteilhaft wählt man eine Verweilzeit zwischen Aminzugabeende und Säurezugabebeginn, die zweckmäßig 1 bis 100, bevorzugt 1 bis 10 Sekunden beträgt.

Man kann die Ausgangsstoffe in stöchiometrischen Mengen oder im Überschuß, zweckmäßig in einem Verhältnis von 1,0 bis 1,5, vorteilhaft 1,1 bis 1,5 Mol Ausgangsstoff III je Mol Ausgangsstoff II, umsetzen. Man verwendet vorteilhaft eine Menge von 1,0 bis 1,6, insbesondere von 1,4 bis 1,5 Äquivalenten Säure, bezogen auf 1 Mol Ausgangsstoff II. Es können anorganische oder organische Säuren verwendet werden. Anstelle einbasischer Säuren können auch äquivalente Mengen mehrbasischer Säuren zur Anwendung gelangen. Beispielsweise sind folgende Säuren geeignet: Chlorwasser-

stoff, Bromwasserstoff, Jodwasserstoff, Schwefelsäure, Phosphorsäure, Kohlensäure, Ameisensäure, Essigsäure, 2-Ethylhexansäure. Die Säuren können in konzentrierter Form, im Gemisch miteinander und/oder mit einem Lösungsmittel, insbesondere Wasser, angewendet werden. Bei verdünnten, wäßrigen Säuren sind 1-bis 98-gewichtsprozentige Säuren, z.B. 10-bis 30-gewichtsprozentige Salzsäure, 10- bis 50-, vorzugsweise 20- bis 30-gewichtsprozentige Schwefelsäure oder 70- bis 85-gewichtsprozentige Ameisensäure, vorteilhaft. Bevorzugt sind Salzsäure, Schwefelsäure, Phosphorsäure, Ameisensäure. Der pH-Wert der Umsetzung beträgt 6 bis 8, vorzugsweise von 6,4 bis 7,6, insbesondere von 6,7 bis 7,3.

Aus dem Reaktionsgemisch wird der Endstoff I in üblicher Weise, z.B. durch Filtration und fraktionierte Destillation, abgetrennt. Die abgeschiedenen Anthranilsäureamide I können direkt abgesaugt werden; die Mutterlauge kann wieder in den Prozeß zurückgeführt werden. Die Ausbeuten an den Amiden I liegen zwischen 80 und 95 % der Theorie; die Reinheit liegt bei 99 % (diazometrisch).

Für bestimmte Anwendungszwecke ist es vorteilhaft, die Amide nicht in fester Form zu isolieren, sondern in Gegenwart eines mit Wasser nicht mischbaren Lösungsmittels zu arbeiten. Dazu wird gleichzeitig mit der Aminzugabe ein organisches Lösungsmittel zudosiert. Besonders geeignet sind hierzu 1,2-Dichlorethan, Dichlorethylen, 1,2-Dichlorpropan, Chlorbenzol, Dichlorbenzol. Eine besonders vorteilhafte Form des neuen Verfahrens ist die kontinuierliche Ausführung. Dazu wird die aus Phthalimid durch Hydrolyse mit NaOH hergestellte Phthalamidsäurelösung in einer Mischvorrichtung kontinuierlich mit Hypohalogeniten umgesetzt. Nach Verweilzeiten von 1 bis 30 Sekunden werden gleichzeitig das Amin, die Mineralsäure und das organische Lösungsmittel zugegeben, ein pH-Wert von 6 bis 8 gehalten und nach Verweilzeiten von

von 1 bis 60 Minuten (bei Temperaturen von 20 bis 80°C) die organische Phase von der wäßrigen Phase getrennt und weiterverarbeitet.

Die nach dem Verfahren der Erfindung herstellbaren Anthranilsäureamide I sind wertvolle Ausgangsstoffe für die Herstellung von Farbstoffen, Riechstoffen und Schädlingsbekämpfungsmitteln. So kann man z.B. durch Umsetzung mit wasserabspaltenden Mitteln 2-Amino-benzonitrile, wertvolle Zwischenprodukte für die Herstellung von Azofarbstoffen, erhalten. Bezüglich der Verwendung wird auf Ullmanns Encyklopädie der technischen Chemie, Band 3, Seiten 310 und 465 ff, Band 19, Seiten 300 ff und auf die vorgenannten Veröffentlichungen verwiesen.

Die in den folgenden Beispielen angeführten Teile bedeuten Gewichtsteile. Sie verhalten sich zu den Volumenteilen wie Kilogramm zu Liter.

Beispiel 1

42 Teile flüssiges Phthalimid werden stündlich in einer Mischvorrichtung kontinuierlich in 48 Teilen 25-gewichtsprozentiger wäßriger Natronlauge und 400 Teilen Wasser gelöst und kontinuierlich bei einer Temperatur von 25°C ein Teil pro Stunde 30-gewichtsprozentige wäßrige Lösung des Natriumsalzes der Amidosulfonsäure zudosiert.

Diese Reaktionslösung wird in einer Mischdüse mit 155 Teilen wäßriger Natriumhypochloritlösung (13,6 Gew.% aktives Chlor; 21 Teile Natriumhypochlorit) und 170 Teilen $H_2O$ bei 25°C gemischt.

Nach einer Verweilzeit von 3 Sekunden in einem Rohrreaktor werden dann stündlich 25 Teile Isopropylamin über eine

Mischdüse zugegeben und nach einer weiteren Sekunde über eine dritte Mischvorrichtung 40 Teile einer 30-gewichtsprozentigen wäßrigen Salzsäurelösung (pH 6,9).

Nach einer Gesamtverweilzeit von 4 Sekunden bei einer Temperatur von 30°C im Rohrreaktor läuft das Reaktionsgemisch in ein Rührgefäß (Verweiler), in den stündlich 235 Teile Dichlorethan einlaufen und gleichzeitig durch Zugabe von weiteren 10 Teilen 30-gewichtsprozentiger wäßriger Salzsäure der pH-Wert bei 6,9 konstant gehalten wird. Die Temperatur im Verweiler beträgt 60°C.

Nach einer Aufenthaltszeit von 10 Minuten im Verweiler wird die organische Phase kontinuierlich über eine Abscheidevorrichtung von der wäßrigen Phase getrennt und weiterverarbeitet.

Man erhält stündlich 46 Teile Anthranilsäureisopropylamid als 12-gewichtsprozentige Lösung in Dichlorethan (90,5 % der Theorie; Festpunkt 149°C, Reinheit 99,3 %, durch Diazotierung bestimmt). Die Raum-Zeit-Ausbeute beträgt 0,2 Teile pro Stunde und Liter.

Beispiel 2

Man führt die Reaktion analog Beispiel 1 aus, aber anstelle des Lösungsmittels Dichlorethan werden stündlich 320 Teile Dichlorpropan eingesetzt, die Temperatur im Verweiler bei 60°C konstant gehalten und die Verweilzeit auf 4 Minuten gegenüber 10 Minuten in Beispiel 1 verkürzt. Die Verweilzeit zwischen Ende der Herstellung des Ausgangsgemisches und dem Zugabebeginn des Ammoniaks/Amins beträgt 3 Sekunden bzw. dem Zugabebeginn der Säure 4 Sekunden.

Man erhält stündlich 45 Teile Anthranilsäureisopropylamid (89 % der Theorie, Reinheit 99 %, durch Diazotierung bestimmt). Die Raum-Zeit-Ausbeute beträgt 0,37 Teile pro Stunde und Liter. Fp 149°C.

Beispiel 3

Analog Beispiel 1 werden 42 Teile Phthalimid, 48 Teile 25-gewichtsprozentiger wäßriger Natronlauge, 400 Teile Wasser, ein Teil einer 30-gewichtsprozentigen wäßrigen Lösung des Natriumsalzes der Amidosulfonsäure, 149 Teile wäßriger Natriumhypochloritlösung (13,6 Gew.% aktives Chlor) umgesetzt.

Anstelle von Isopropylamin werden stündlich 20 Teile einer 25-gewichtsprozentigen wäßrigen Ammoniaklösung zugemischt und bei einer Temperatur von 50°C 40 Teile einer 30-gewichtsprozentigen wäßrigen Salzsäurelösung. Die Verweilzeit zwischen Ende der Herstellung des Ausgangsgemisches und dem Zugabebeginn des Ammoniaks beträgt 15 Sekunden bzw. dem Zugabebeginn der Säure 35 Sekunden.

Nach einer Gesamtverweilzeit von 40 Sekunden läuft das Reaktionsgemisch in einen Verweiler, in dem durch Zugabe von weiteren 12 Teilen 30-gewichtsprozentiger wäßriger Salzsäure der pH-Wert bei 6,9 konstant gehalten wird. Die Temperatur im Verweiler beträgt 50°C. Nach einer Verweilzeit von 13 Minuten wird das gebildete Anthranilsäureamid aus der wäßrigen Lösung mittels einer Extraktionskolonne mit Dichlorethan ausgezogen und weiterverarbeitet.

Man erhält stündlich 34 Teile Anthranilsäureamid (84 % der Theorie) vom Fp 110°C, Reinheit 99,5 % (diazometrisch bestimmt).

Beispiel 4

21,6 Teile 4-Chlorphthalamidsäure werden in 100 Teilen Wasser und 17 Teilen 25-gewichtsprozentiger wäßriger Natronlauge gelöst und 0,1 Teil Kaliumiodid zugefügt. Man kühlt die Lösung auf 15°C ab und gibt noch 57 Teile einer wäßrigen Natriumhypochloritlösung von ebenfalls 15°C zu (enthaltend 8,0 Teile Natriumhypochlorit).

Nach 30 Sekunden werden 9 Teile Isopropylamin und 8 Teile einer 30-gewichtsprozentigen wäßrigen Salzsäure zugefügt und der pH-Wert bei pH 7,0 konstant gehalten (durch Zugabe weiterer Salzsäure). Die Verweilzeit zwischen Ende der Herstellung des Ausgangsgemisches und dem Zugabebeginn des Amins beträgt 30 Sekunden bzw. dem Zugabebeginn der Säure 31 Sekunden. Man rührt bei 30°C insgesamt eine halbe Stunde nach, filtriert und trocknet.

Die Ausbeute beträgt 17 Teile 4-Chloranthranilsäureisopropylamid, entsprechend 82 % der Theorie, Fp 168 - 170°C.

## Patentanspruch

Verfahren zur Herstellung von Anthranilsäureamiden der Formel

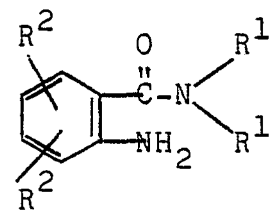

I,

worin die einzelnen Reste $R^1$ und $R^2$ gleich oder verschieden sein können und jeweils Wasserstoff oder einen aliphatischen Rest bedeuten, $R^1$ auch einen cycloaliphatischen, araliphatischen, aromatischen Rest bezeichnet, $R^2$ auch für ein Halogenatom oder eine Alkoxygruppe stehen kann, dadurch gekennzeichnet, daß man Phthalamidsäuren der Formel

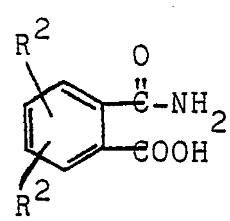

II,

worin $R^2$ die vorgenannte Bedeutung besitzt, mit Hypohalogeniten und Ammoniak oder Aminen der Formel

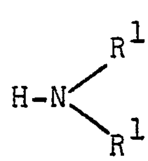

III,

worin $R^1$ die vorgenannte Bedeutung besitzt, umsetzt, wobei man den Ausgangsstoff II in wäßriger Lauge löst, mit Amidosulfonsäure, Sulfamid und/oder Kaliumiodid als Katalysator und Hypohalogenit mischt, die Mischung 1 bis 1 000 Sekunden stehen läßt und nun das Amin und darauf Säure bis zu einem pH-Wert von 6 bis 8 in das Gemisch eingibt.

0057424

Europäisches Patentamt

**EUROPÄISCHER RECHERCHENBERICHT**

Nummer der Anmeldung
EP 82100566.7

| EINSCHLÄGIGE DOKUMENTE | | | KLASSIFIKATION DER ANMELDUNG (Int. Cl.3) |
|---|---|---|---|
| Kategorie | Kennzeichnung des Dokuments mit Angabe, soweit erforderlich, der maßgeblichen Teile | betrifft Anspruch | |
| X | BEILSTEINS HANDBUCH DER ORGANISCHEN CHEMIE, 4. Auflage, Band XXVII, 1937<br>JULIUS SPRINGER, Berlin<br>Seite 264<br>-- | 1 | C 07 C 103/28<br>C 07 C 103/76<br>C 07 C 102/00 |
| D,X | DE - A1 - 2 719 020 (BAYER)<br>* Patentanspruch 1 *<br>---- | 1 | |

RECHERCHIERTE SACHGEBIETE (Int. Cl.3)

C 07 C 102/00
C 07 C 103/00

KATEGORIE DER GENANNTEN DOKUMENTE

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze
E: älteres Patentdokument, das jedoch erst am oder nach dem Anmeldedatum veröffentlicht worden ist
D: in der Anmeldung angeführtes Dokument
L: aus andern Gründen angeführtes Dokument

&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| WIEN | 14-04-1982 | HOFBAUER |

EPA form 1503.1 06.78